# EUROPÄISCHE PATENTANMELDUNG

(11) **EP 3 413 342 A1**
(43) Veröffentlichungstag der Anmeldung: **12.12.2018**
(21) Anmeldenummer: 17174971.6
(22) Anmeldetag: 08.06.2017
(51) Int. Cl.: H01L 23/00, H01L 23/498

(54) **ELEKTRONISCHE BAUGRUPPE UND VERFAHREN ZUR HERSTELLUNG EINER SOLCHEN**

(71) Anmelder: Dyconex AG, 8303 Bassersdorf (CH)
(72) Erfinder: Hauer, Marc, 8610 Uster (CH); Bihler, Eckardt, 8406 Winterthur (CH); Held, Jochen, 6415 Arth (CH)
(74) Vertreter: Randoll, Sören

(57) **Zusammenfassung**

Elektronische Baugruppe auf einem flexiblen flächigen Schaltungssubstrat mit einer Leiterkonfiguration auf einer ersten Substratoberfläche und mehreren elektronischen Bauelementen auf der gegenüberliegenden zweiten Substratoberfläche, wobei die Bauelemente Bauelementkontakte aufweisen, die über Durchkontaktierungen in dem Schaltungssubstrat und die Leiterkonfiguration selektiv elektrisch angeschlossen sind, wobei das Schaltungssubstrat ein thermoplastisches Polymer aufweist und die Bauelementkontakte im Bereich der Durchkontaktierungen in die zweite Substratoberfläche eingeschmolzen bzw. thermisch eingepresst sind.

## Beschreibung

Die Erfindung betrifft eine elektronische Baugruppe auf einem flexiblen flächigen Schaltungssubstrat mit einer Leiterkonfiguration auf einer ersten Substratoberfläche und mehreren elektronischen Bauelementen auf der gegenüberliegenden zweiten Substratoberfläche, wobei die Bauelemente Bauelementkontakte aufweisen, die über Durchkontaktierungen in dem Schaltungssubstrat und die Leiterkonfiguration selektiv elektrisch angeschlossen sind. Sie betrifft des Weiteren ein Verfahren zur Herstellung einer solchen Baugruppe.

Elektronische Baugruppen, die auf flächigen Schaltungssubstraten mir vorgefertigter Leiterkonfiguration, sog. Leiterplatten, aufgebaut sind, sind seit Jahrzehnten aus der Elektronik und Elektrotechnik nicht mehr wegzudenken und sind Gegenstand ständiger Weiterentwicklung.

Von vielen Weiterentwicklungen haben in den letzten Jahren die sog. System-In-Package(SIP)-Lösungen und unter diesen speziell die Embedded Chips oder Chip-In-Polymer-Anordnungen besondere Aufmerksamkeit gefunden. Zum Stand der Technik auf diesem speziellen Gebiet verweisen wir auf L. Boettcher et al. "Embedding of Chips for System in Package realization - Technology and Applications", www.izm.fraunhofer.de, mit weiteren Literaturhinweisen. Bei dieser Technologie können, wie in der genannten Veröffentlichung angegeben, Standard-Leiterplattenaufbauten genutzt werden; bekannt ist auch der Einsatz von Schaltungssubstraten, die im Wesentlichen aus einem duroplastischen Polymer bestehen.
Es hat sich gezeigt, dass Standard-Leiterplattensubstrate speziell in aggressiven Umgebungen (etwa aggressiven atmosphärischen Umgebungen oder dem Körper von Lebewesen) chemisch nicht inert genug sind und/oder zu hohe Diffusionsraten für Sauerstoff bzw. Wasser haben. Die Anwendungsbereiche der genannten Technologie in Verbindung mit Standard-Leiterplatten sind daher in dieser Hinsicht begrenzt, und es wird nach alternativen Materialien gesucht. Bekannt ist hierbei, dass bestimmte thermoplastische Polymere insbesondere hinsichtlich niedriger Diffusionsraten von Sauerstoff, Wasser und Ionen vorteilhafte Eigenschaften haben; derartige Polymere sind aber beim thermischen Laminieren in der xy-Ebene nicht sehr stabil. Deshalb zieht der Fachmann derartige Schaltungssubstrat-Materialien im Hinblick auf die hohen Stabilitätsanforderungen des Substrats bei der Positionierung vieler mikroelektronischer Schaltkreise oder anderer elektronischer Bauelemente auf einem einzelnen Substrat üblicherweise nicht in Betracht.

Aufgabe der Erfindung ist es, eine verbesserte elektronische Baugruppe, die insbesondere mit einer hohen Positionsgenauigkeit der eingesetzten Bauelemente gefertigt werden kann und für den Einsatz in implantierbaren Geräten sowie in für aggressive Umgebungen geeigneten Geräten optimiert ist, sowie ein Verfahren zu deren Herstellung anzugeben.

Diese Aufgabe wird in ihrem Vorrichtungsaspekt durch eine elektronische Baugruppe mit den Merkmalen des Anspruchs 1 und in ihrem Verfahrensaspekt durch ein Verfahren mit den Merkmalen des Anspruchs 10 gelöst. Zweckmäßige Fortbildungen des Erfindungsgedankens sind Gegenstand der jeweiligen abhängigen Ansprüche.

Die Erfindung beinhaltet eine bewusste Abkehr von der allgemein geltenden Einschätzung, dass thermoplastische Polymere als Substratmaterial des Schaltungssubstrat elektronischer Baugruppen aus mechanischen Gründen nicht geeignet sind, und sie schließt weiterhin strukturelle wie auch technologische Aspekte ein, mit denen eine elektronische Baugruppe unter Einsatz eines derartigen Schaltungssubstrats unter Erfüllung aller Anforderungen realisiert werden kann.

Die Merkmale der erfindungsgemäßen Baugruppe sind im Sinne des weit aufgefächerten Standes der Technik mit dem breitestmöglichen Bedeutungsgehalt zu verstehen. Obgleich das Vorliegen einer solchen Leiterkonfiguration auf der ersten Substratoberfläche wesentlich ist, kann auch auf der zweiten Substratoberfläche eine Leiterkonfiguration angeordnet sein, und auch diese kann in der soeben genannten Weise ausgestaltet sein. Ähnlich können neben den erfindungsgemäß zumindest auf der zweiten Oberfläche des Schaltungssubstrats angeordneten Bauelementen auch auf der ersten Substratoberfläche Bauelemente platziert sein. Grundsätzlich ist auch eine mehrschichtige Ausführung der Baugruppe mit mehreren Schaltungssubstraten und jeweils zugeordneten ersten und zweiten Oberflächen und entsprechend positionierten Leiterkonfigurationen und Bauelementgruppen als im Rahmen der Erfindung liegend zu verstehen.

Die Durchkontaktierungen (Vias) umfassen in Durchbrüchen des Schaltungssubstrats realisierte Metallisierungen. Die Durchbrüche werden im Folgenden als "Bohrungen" bezeichnet, auch wenn sie nicht mittels eines Bohrwerkzeuges erzeugt werden, und obwohl sie nicht notwendigerweise kreiszylindrisch ausgeführt sind. In einem vorgefertigten Muster von Durchbrüchen bzw. "Bohrungen" kann in Ausführungen der Erfindung, entsprechend dem Bestückungsplan des Schaltungssubstrats nur eine vorbestimmte Teilmenge mit einer Metallisierung versehen sein. Solche Ausführungen sind mit einer selektiven Metallisierungstechnik (etwa mittels einer Metallisierungs-Maske) realisierbar. Alternativ können auch sämtliche Bohrungen des Bohrungsmusters mit einer Innenwand-Metallisierung versehen sein, was den zugehörigen Metallisierungsprozess vereinfacht und weniger fehleranfällig macht.

In einer Ausführung der Erfindung sind die Leiterkonfiguration aus einer ersten Metallisierungsschicht und die Durchkontaktierungen aus einer zweiten Metallisierungsschicht gebildet. Dies bedeutet, dass die Grund-Verbindungsstruktur auf dem Schaltungssubstrat vorgefertigt und die Verbindung zwischen den eingesetzten Bauelementen und jener Grundstruktur nach dem Anfügen bzw. Einfügen der Bauelemente an/in das Schaltungssubstrat in einem späteren getrennten Schritt erzeugt werden kann.

In bevorzugten Ausführungen weisen die Durchkontaktierungen eine in Bohrungen des Schaltungssubstrats abgeschiedene Metallisierung auf. Bedarfsweise ist hierbei die abgeschiedene Metallisierung galvanisch verstärkt. In weiteren Ausgestaltungen dieser Ausführungen weisen die Durchkontaktierungen mindestens ein Metall aus der Gruppe Kupfer, Gold, Titan, Wolfram, Palladium Chrom, Nickel auf.

In einer weiteren vorteilhaften Ausführung ist die elektronische Baugruppe mit einer mindestens eines der Bauelemente einkapselnden thermoplastischen Schutzfolie mindestens auf der zweiten Substratoberfläche versehen. Diese kann aus dem gleichen Material wie das Schaltungssubstrat bestehen, je nach Anwendungssituation ist aber auch der Einsatz eines anderen thermoplastischen Materials möglich. In aus derzeitiger Sicht vorteilhaften Ausführungen weist das oder ein thermoplastisches Material mindestens ein Material aus der Gruppe flüssigkristallines Polymer, LCP; Polyetherketon, PEEK; fluorierte Polymere, oder Polyurethane auf.

In weiteren Ausführungen weist das Schaltungssubstrat eine Dicke im Bereich zwischen 10 µm und 500 µm, insbesondere zwischen 10 µm und 100 µm, auf. Je nach Größe und Komplexität der elektronischen Baugruppe und der Anwendungssituation kommen grundsätzlich aber auch dünnere oder dickere Substrate in Betracht.

In aus derzeitiger Sicht besonders vorteilhaften Funktionen/Anwendungen ist die elektronische Baugruppe ausgebildet als implantierbares elektromedizinisches Implantat oder Baugruppe eines solchen. Es kann sich aber auch speziell um ein Gerät oder eine Baugruppe eines Gerätes handeln, das/die unter "rauen" Umgebungsbedingungen, insbesondere in chemisch aggressiven Umgebungen, einzusetzen ist.

Das erfindungsgemäße Verfahren zur Herstellung der vorab erläuterten Baugruppe umfasst zumindest die folgenden Schritte:
- Bereitstellen des ein thermoplastisches Polymer aufweisenden flexiblen flächigen Schaltungssubstrats,
- Erzeugen der Leiterkonfiguration auf der ersten Substratoberfläche,
- Erzeugen eines Bohrungsmusters entsprechend der Geometrie der Leiterkonfiguration,
- Erhitzen der auf die zweite Substratoberfläche aufzusetzenden Bauelemente,
- mit dem Bohrungsmuster im Schaltungssubstrat ausgerichtetes Aufsetzen der Bauelemente auf die zweite Substratoberfläche mit vorbestimmtem Anpressdruck und hierdurch bewirktes Einschmelzen bzw. thermisches Einpressen der Bauelementkontakte in die zweite Substratoberfläche im Bereich der Bohrung,
- selektive Metallabscheidung in das Bohrungsmuster und auf die Umgebung der Bohrungen von der ersten Substratoberfläche aus zur Erzeugung der Durchkontaktierungen.
Diese Schritte werden bevorzugt, aber nicht für jeden Schritt notwendigerweise, auch in der genannten Reihenfolge ausgeführt. Unter einer Leiterkonfiguration ist im allgemeinen Sinne ein geometrisches Muster mit im Wesentlichen linearen, möglicherweise aber auch flächigen leitfähigen Beschichtungsabschnitten zu verstehen, in das auch passive Funktionselemente, wie Widerstände, Induktivitäten und Kapazitäten, eingefügt sein können.

Weiterhin ist im Schritt, der dem Fixieren der Bauelemente im Schaltungssubstrat dient, statt eines Erwärmens der Bauelemente grundsätzlich auch eine Erwärmung des Schaltungssubstrats als solchen oder eine Kombination der Erwärmung sowohl der Bauelemente als auch des Schaltungssubstrats mit jeweils geeigneten Mitteln möglich.

In einer Ausführung der Erfindung werden die Bauelemente auf eine Aufsetztemperatur im Bereich zwischen 100°C und 400°C, insbesondere zwischen 250°C und 350°C erwärmt, und/oder der Anpressdruck liegt im Bereich zwischen 0,05N und 50N, insbesondere zwischen 0,1N und 30N.

In vorteilhaften Ausführungen des Verfahrens wird die Metallabscheidung in das Bohrungsmuster nach dem Einschmelzen bzw. thermischen Einpressen der Bauelemente, insbesondere mittels eines elektrochemischen oder physikalischen Dünnschichtverfahrens, CVD bzw. PVD, ausgeführt. Es können hier herkömmliche, etablierte Abschaltungsverfahren eingesetzt werden, soweit nicht mit Blick auf das thermoplastische Substratmaterial bestimmte Einschränkungen erforderlich sind.

Optional wird nach der Metallabscheidung eine galvanische Verstärkung der abgeschiedenen Dünnschicht ausgeführt. Die Notwendigkeit des Ausführens eines solchen weiteren (und hinsichtlich der Prozessbedingungen unter Umweltschutz- und Arbeitsschutzaspekten nicht unkritischen) Verfahrensschrittes ist eine Entscheidung aufgrund der erforderlichen Leitfähigkeit und Robustheit der Leiterkonfiguration bzw. Durchkontaktierungen zu treffen.

In einer aus derzeitiger Sicht bevorzugten Ausführung wird mindestens auf Bereiche der zweiten Substratoberfläche eine thermoplastische Schutzfolie auflaminiert. Dieses Auflaminieren kann im gleichen Schritt wie das Bestücken des Schaltungssubstrats mit den Bauelementen erfolgen, also gewissermaßen indem die Bauelemente beim Auflaminieren der Schutzfolie in das Schaltungssubstrat gepresst werden. Grundsätzlich ist aber auch eine Trennung beider Schritte möglich. Neben der zweiten Substratoberfläche kann auch die erste Substratoberfläche, also diejenige mit der Leiterkonfiguration, mit einer Schutzfolie bedeckt werden, so dass insbesondere eine beidseitige Versiegelung zum optimalen Schutz der elektronischen Baugruppe vor Umgebungseinflüssen realisiert wird.

Hinsichtlich der Verfahrensbedingungen, speziell Temperatur- und Anpressdruck, gelten die obigen Feststellungen für den Schritt des Bestückens des Schaltungssubstrats mit den Bauelementen normalerweise analog; beim Einsatz spezieller Laminierungsfolien sind aber ggfs. auch andere Verfahrensparameter einzustellen.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine schematische Querschnittsdarstellung eines Ausführungsbeispiels der erfindungsgemäßen elektronischen Baugruppe während des Herstellungsprozesses und
- Fig. 2: eine schematische Darstellung der Baugruppe nach Fig. 1 nach Fertigstellung.

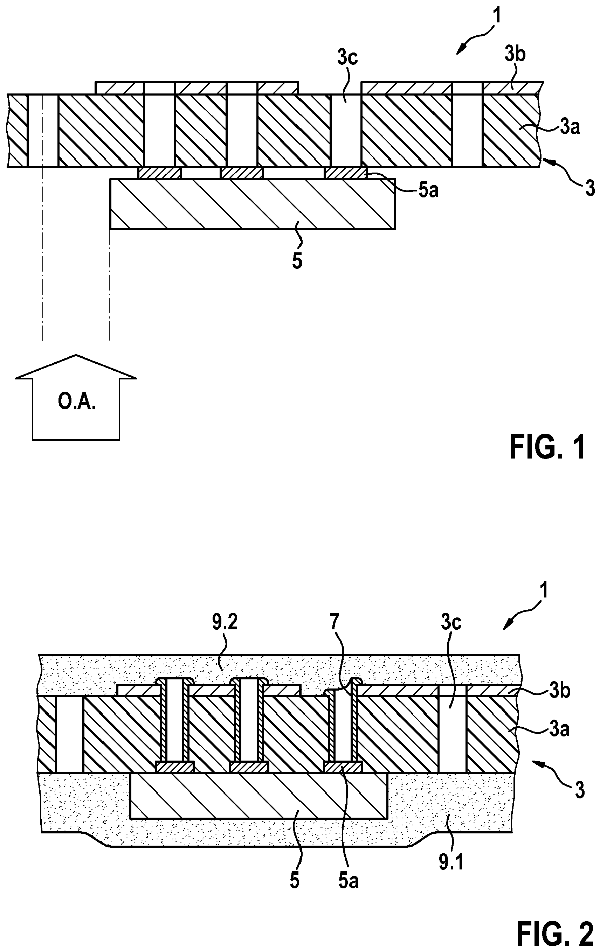

Fig. 1 zeigt schematisch ein Schaltungssubstrat 3 einer (unfertigen) elektronischen Baugruppe 1, das aus einer LCP(flüssigkristallinen Polymer)-Folie 3a mit einer einseitig aufgeschichteten Leiterkonfiguration 3b besteht, zusammen mit einem Mikrochip 5, der auf einer Hauptoberfläche Bauelementkontakte 5a trägt. Der Mikrochip 5 ist auf der der Leiterkonfiguration 3b gegenüberliegenden Oberfläche des flexiblen flächigen Schaltungssubstrats 3 derart aufgesetzt, dass die Bauelementkontakte 5a mit einem Muster von Bohrungen 3c im Schaltungssubstrat 3 ausgerichtet sind.

Die Ausrichtung zwischen dem Bohrungsmuster im Schaltungssubstrat 3 und dem Mikrochip 5 wird mittels eines optischen Ausrichtungsverfahrens (Optical Alignement) ausgeführt, welches in der Figur durch einen Pfeil mit dem Bezugszeichen O.A. symbolisiert ist. Für diesen Schritt können branchentypische Montagesysteme wie Bilderfassung verwendet werden. Dabei werden die Bohrungen 3c im Schaltungssubstrat (der LCP-Folie) optisch aufgenommen und die Bauelemente mit geringer Lagetoleranz (ca. 5-25 µm) auf dem Schaltungssubstrat positioniert.

Bei der Positionierung wird der Mikrochip 5 auf eine Temperatur in der Nähe des Tg der LCP-Folie 3a, z.B. einen Wert im Bereich zwischen 250°C und 350°C, erwärmt und mit vorbestimmter Kraft, beispielsweise im Bereich zwischen 0,1N und 30N, in die benachbarte Oberfläche der LCP-Folie gedrückt. Diese wird dabei lokal erwärmt und damit erweicht, so dass die Bauelementkontakte 5a sich in die benachbarte Umgebung der Bohrungen 3c eindrücken und das Bauelement spaltfrei bündig an der benachbarten Oberfläche des Schaltungssubstrats 3 haftet.

Fig. 2 zeigt den Fertigstellungszustand der Baugruppe 1, in dem das erwähnte Anhaften des Mikrochips 5 am Schaltungssubstrat bei in die Oberfläche eingedrungenen Bauelementkontakten 5a gegeben und in der Figur zu erkennen ist. Des Weiteren ist in diesem Zustand eine Metallisierung mit der dem Mikrochip 5 gegenüberliegenden Oberfläche des Schaltungssubstrats 3 selektiv in denjenigen Bohrungen 3c erfolgt, in denen Bauelementkontakte 5a sitzen. Hierdurch sind Durchkontaktierungen 7 gebildet, die sich als Innenwand-Metallisierung der entsprechenden Bohrungen 3c von den Bauelementkontakten 5 bis auf die Leiterkonfiguration 3b erstrecken und so die Bauelementkontakte mit der Leiterkonfiguration elektrisch verbinden. Diese Innenwand-Metallisierung kann durch ein elektrochemisches oder physikalisches Dünnfilmverfahren oder auch eine Kombination solcher Verfahren erzeugt werden.

Anschließend wurde auf beide Oberflächen des Schaltungssubstrats 3 jeweils eine Schutzfolie 9.1 bzw. 9.2 auflaminiert. Auch die Schutzfolien 9.1, 9.2 bestehen aus einem thermoplastischen Material, beispielsweise einer mit der LCP-Folie 3a gleichartigen LCP-Folie. Diese verkapselt die elektronische Baugruppe 1 weitgehend diffusionsdicht gegenüber der Umgebung und ermöglicht so den Einsatz in medizinischen Implantaten oder Geräten zum Einsatz in rauen Umgebungen.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Elektronische Baugruppe (1) auf einem flexiblen flächigen Schaltungssubstrat (3) mit einer Leiterkonfiguration (3b) auf einer ersten Substratoberfläche und mehreren elektronischen Bauelementen (5) auf der gegenüberliegenden zweiten Substratoberfläche, wobei die Bauelemente Bauelementkontakte (5a) aufweisen, die über Durchkontaktierungen (7) in dem Schaltungssubstrat (3) und die Leiterkonfiguration (3b) selektiv elektrisch angeschlossen sind,
wobei das Schaltungssubstrat (3) ein thermoplastisches Polymer aufweist und die Bauelementkontakte (5a) im Bereich der Durchkontaktierungen (7) in die zweite Substratoberfläche eingeschmolzen bzw. thermisch eingepresst sind.

2. Elektronische Baugruppe (1) nach Anspruch 1, wobei die Leiterkonfiguration (3b) aus einer ersten Metallisierungsschicht und die Durchkontaktierungen (7) aus einer zweiten Metallisierungsschicht gebildet sind.

3. Elektronische Baugruppe (1) nach einem der vorangehenden Ansprüche, wobei die Durchkontaktierungen (7) eine in Bohrungen (3c) des Schaltungssubstrats (3) abgeschiedene Metallisierung aufweisen.

4. Elektronische Baugruppe (1) nach Anspruch 3, wobei die abgeschiedene Metallisierung galvanisch verstärkt ist.

5. Elektronische Baugruppe (1) nach Anspruch 3 oder 4, wobei die Durchkontaktierungen (7) mindestens ein Metall aus der Gruppe Kupfer, Gold, Titan, Wolfram, Palladium Chrom, Nickel aufweisen.

6. Elektronische Baugruppe (1) nach einem der vorangehenden Ansprüche, mit einer mindestens eines der Bauelemente (5) einkapselnden thermoplastischen Schutzfolie (9.1, 9.2) mindestens auf der zweiten Substratoberfläche.

7. Elektronische Baugruppe (1) nach einem der vorangehenden Ansprüche, wobei das oder ein thermoplastisches Material mindestens ein Material aus der Gruppe flüssigkristallines Polymer, LCP; Polyetherketon, PEEK; fluoriertes Polymer oder Polyurethan aufweist.

8. Elektronische Baugruppe (1) nach einem der vorangehenden Ansprüche, wobei das Schaltungssubstrat (3) eine Dicke im Bereich zwischen 10 µm und 500 µm, insbesondere zwischen 10 µm und 100 µm, aufweist.

9. Elektronische Baugruppe (1) nach einem der vorangehenden Ansprüche, ausgebildet als implantierbares elektromedizinisches Implantat oder Baugruppe eines solchen.

10. Verfahren zur Herstellung einer elektronischen Baugruppe nach einem der vorangehenden Ansprüche, mit den Schritten:
- Bereitstellen des ein thermoplastisches Polymer aufweisenden flexiblen flächigen Schaltungssubstrats (3),
- Erzeugen der Leiterkonfiguration (3b) auf der ersten Substratoberfläche,
- Erzeugen eines Bohrungsmusters entsprechend der Geometrie der Leiterkonfiguration (3b),
- Erhitzen der auf die zweite Substratoberfläche aufzusetzenden Bauelemente (5),
- mit dem Bohrungsmuster im Schaltungssubstrat (3) ausgerichtetes Aufsetzen der Bauelemente (5) auf die zweite Substratoberfläche mit vorbestimmtem Anpressdruck und hierdurch bewirktes Einschmelzen bzw. thermisches Einpressen der Bauelementkontakte in die zweite Substratoberfläche im Bereich der Bohrung (3c),
- selektive Metallabscheidung in das Bohrungsmuster und auf die Umgebung der Bohrungen (3c) von der ersten Substratoberfläche aus zur Erzeugung der Durchkontaktierungen (7).

11. Verfahren nach Anspruch 10, wobei die Bauelemente (5) auf eine Aufsetztemperatur im Bereich zwischen 100°C und 400°C, insbesondere zwischen 250°C und 350°C erwärmt werden und/oder der Anpressdruck im Bereich zwischen 0,05N und 50N, insbesondere zwischen 0,1N und 30N liegt.

12. Verfahren nach Anspruch 10 oder 11, wobei die Metallabscheidung in das Bohrungsmuster nach dem Einschmelzen bzw. thermischen Einpressen der Bauelemente (5), insbesondere mittels eines elektrochemischen oder physikalischen Dünnschichtverfahrens, CVD bzw. PVD, ausgeführt wird.

13. Verfahren nach Anspruch 12, wobei nach der Metallabscheidung eine galvanische Verstärkung der abgeschiedenen Dünnschicht ausgeführt wird.

14. Verfahren nach einem der Ansprüche 10 bis 13, wobei mindestens auf Bereiche der zweiten Substratoberfläche eine thermoplastische Schutzfolie (9.1, 9.2) auflaminiert wird.

15. Verfahren nach Anspruch 14, wobei das Auflaminieren der Schutzfolie (9.1, 9.2) bei einer Temperatur im Bereich zwischen 100°C und 400°C, insbesondere zwischen 250°C und 350°C erfolgt und/oder der Anpressdruck im Bereich zwischen 0,05N und 50N, insbesondere zwischen 0,1N und 30N liegt.
